# EUROPÄISCHE PATENTSCHRIFT

(11) **EP 2 976 046 B1**
(45) Veröffentlichungstag und Bekanntmachung des Hinweises auf die Patenterteilung: **13.03.2019**
(21) Anmeldenummer: 14712603.1
(22) Anmeldetag: 21.03.2014
(51) Int. Cl.: A61F 2/44, A61F 2/46

(54) **INSTRUMENTENSET ZUM EINBRINGEN EINES KÖRBCHENS IN DAS BANDSCHEIBENFACH ZWISCHEN ZWEI WIRBELKÖRPERN**
INSTRUMENT SET FOR INTRODUCING A CAGE INTO THE INTERVERTEBRAL DISC SPACE
ENSEMBLE D'INSTRUMENTS POUR INTRODUIRE UNE CAGE DANS UN ESPACE INTERVERTÉBRAL ENTRE DEUX CORPS VERTÉBRAUX

(30) Priorität: 22.03.2013 DE 102013004964; 14.08.2013 DE 202013007361 U
(43) Veröffentlichungstag der Anmeldung: 27.01.2016
(73) Patentinhaber: Joimax GmbH, 76227 Karlsruhe (DE)
(72) Erfinder: RIES, Wolfgang, 76351 Linkenheim-Hochstetten (DE)
(74) Vertreter: Lichti - Patentanwälte Partnerschaft mbB
(86) Internationale Anmeldenummer: PCT/EP2014/000779
(87) Internationale Veröffentlichungsnummer: WO 2014/146797

(56) Entgegenhaltungen:
- EP-A1- 0 077 159
- EP-A2- 1 634 549
- WO-A1-01/60263
- WO-A1-2011/094748
- DE-A1-102008 045 174
- US-A1- 2001 016 741
- US-A1- 2004 106 997
- US-A1- 2005 251 257
- US-A1- 2006 074 488
- US-A1- 2007 021 835
- US-A1- 2007 106 319
- US-A1- 2007 162 138
- US-A1- 2007 213 826
- US-A1- 2009 138 043
- US-A1- 2010 318 028
- US-A1- 2011 319 995
- US-A1- 2012 078 315
- US-A1- 2013 006 362

## Beschreibung

Die Erfindung betrifft ein Instrumentenset zum Einbringen eines Körbchens in das Bandscheibenfach zwischen zwei Wirbelkörper nach dem Oberbegriff des Anspruchs 1.

Bei einer Reihe von Wirbelsäulenschäden, insbesondere Bandscheibenschäden, wie Wirbelgleiten und Instabilität nach einem Bandscheibenvorfall und Stenose, wird eine Versteifung der Wirbelsäule zwischen den beiden von dem Schaden betroffenen Wirbelkörpern durchgeführt. Hierzu wird ein (Zwischenwirbel-)Körbchen engl.: (Interbody)(Cage) zwischen die Wirbelkörper in das ausgeräumte Bandscheibenfach eingebracht, die Wirbelkörper verwachsen mit dem Körbchen. Dies wird als z.B. lumbale Wirbelfusion (Lumbar Interbody Fusion - LIF) bezeichnet. Für die Brust- und Halswirbelsäule gibt es ähnliche Verfahren. Hierzu wird in der Regel ein Hautschnitt durchgeführt, um unter Sicht zum Bandscheiben-fach vordringen zu können, wobei dies mit Muskel-Destruktion verbunden ist. Dabei können zusätzlich in minimal-invasiver Spinalchirurgie eingebettete perkutane Verschraubungen mit Schrauben-Stab-Systemen zur Gesamtstabilisierung beitragen.

Die bekannten Zugangstechniken zum Einbringen von Körbchen in das Bandscheibenfach zwischen Wirbelkörpern sind mit Nachteilen und Gefahren behaftet.

Die US 2004/0106997 A1 betrifft ein Verfahren und eine Vorrichtung zum Schaffen eines weniger belastenden chirurgischen Wegs zum Zwischenwirbelbereich, um dort ein Zwischenwirbel-Körbchen (Cage) einzusetzen. Die den Arbeitszugang freihaltende Führungs- oder Arbeitshülse kann nur unter einer festen Ausrichtung angesetzt werden, die bestimmt ist durch die Stirnseitenausgestaltung der Führungshülse (im dargestellten Ausführungsbeispiel nicht senkrecht zur Längsachse, sondern leicht abgeschrägt) und die Kontur der Wirbelkörper, die den Wirbelzwischenraum, in dem das Körbchen eingesetzt werden soll, begrenzen und damit durch den Ansatz an den Wirbelkörpern und eben die stirnseitige Ausgestaltung der Führungshülse fest vorgegeben ist. Darüber hinaus ist der Zugang nur in einer Richtung möglich, die der Ebene des Zwischenwirbelbereichs entspricht, also senkrecht zur Wirbelsäulenachse verläuft. Eine Ausrichtung der Führungshülse bzw. ihres größten Abschnitts mit einem Winkel ungleich 90° zur Wirbelsäulenachse ist nicht möglich und damit ein proximaler Eintritt in die Führungshülse außerhalb der Ebene des zu bearbeitenden Zwischenwirbelbereichs ebenfalls nicht. Damit ist der Zugang und die gesamte durch die Vorrichtung der Druckschrift bestimmte Operationstechnik sehr eingeschränkt.

Der Erfindung liegt die Aufgabe zugrunde, ein Instrumentenset und ein Verfahren zum Einbringen eines Körbchens in das Bandscheibenfach bereitzustellen, mit denen in einfacher Weise, unkompliziert und möglichst geringer Gefahr von Beeinträchtigungen des Patienten das Körbchen in das Bandscheibenfach eingebracht werden kann.

Erfindungsgemäß wird die genannte Aufgabe mit einem Instrumentenset der eingangs genannten Art gelöscht, welches die kennzeichnenden Merkmale des Anspruchs 1 aufweist.

Grundsätzlich bestehen alle Teile des erfindungsgemäßen Instrumentensets, insbesondere die in den Körper eindringenden, aus Metall, insbesondere - rostfreiem - Edelstahl.

In bevorzugten Ausgestaltungen ist vorgesehen, dass die Arbeitshülse ist derart in ihrem distalen Bereich flexibel ausgebildet ist und/oder dass die Arbeitshülse als Keilhülse ausgebildet. Die Erfindung sieht damit auch vor, dass die Arbeitshülse des Instrumentensets die nach Aufweitung des Zugangswegs zum Arbeitsort, dem Bandscheibenfach, liegt, einerseits dort verankerbar ist und derart an den beiden das Bandscheiben-fach oberhalb und unterhalb begrenzenden Wirbeln ein Wider-lager findet und damit axial festgelegt ist, andererseits ihre Ausrichtung - insbesondere ihres proximalen Endes - relativ zum Bandscheibenfach bzw. zu dem dieses begrenzenden Wirbeln, oder der Wirbelsäule, insbesondere zu deren Erstreckungsrichtung, variabel ausrichtbar und so in gewissem Umfang beweglich ist.

Die Arbeitshülse ist - wie gesagt - in ihrem distalen Mantelbereich mit Schlitzen mit dazwischen angeordneten Rippen versehen; die Schlitze verleihen dem distalen Endbereich der Arbeitshülse eine Elastizität und Flexibilität, bei noch hinreichender Steifigkeit. Die Schlitze verlaufen insbesondere um den Mantel der Arbeits-hülse entlang von Schraubenlinien, also helikal oder schraubenlinienförmig. Darüber hinaus zeichnet sich eine bevorzugte Ausgestaltung des erfindungsgemäßen Instrumenten-sets dadurch aus, dass auf einer Schraubenlinie mindestens zwei Schlitze durch einen Steg getrennt hintereinander fluchtend angeordnet sind, wobei weiterhin die Breiten von Schlitzen, Rippen und Stegen zwischen 0,5 mm und 2 mm liegen.

Die Schlitze verlaufen von ihrem proximalen Ende schraubenlinienförmig zu ihrem distalen Ende von links nach rechts oder im Uhrzeigersinn bei Blick vom proximalen Ende der Hülse her. Hierdurch wird ein leichtes Einführen der Arbeitshülse unter Drehen entgegen dem Uhrzeigersinn ermöglicht. Zum Schluss erfolgt eine Festlegung der Hülse durch Drehen im Uhrzeigersinn, wodurch ein "Andocken" bzw. ein Festlegen durch umgebende Muskelfasern bewirkt wird. Während des Arbeitens können dabei Muskelfasern, aber auch Nerven, die durch die Schlitze ins Innere der Hülse eindringen sowie Blutungen endoskopisch festgestellt werden.

Bevorzugte Ausgestaltungen des Instrumentensets sehen weiterhin vor, dass das Körbchen und ein Einsetzinstrument zum Einbringen des Körbchens jeweils miteinander zusammenwirkende Verriegelungselemente aufweisen, die eine gemeinsame Verriegelung bilden, wobei insbesondere die Verriegelung von Einsetzinstrument und Körbchen eine angulare Beweglichkeit beider Teile erlaubt.

In weiterer Ausgestaltung ist vorgesehen, dass das Körbchen an einer Stirnseite als Verriegelungselement eine Öffnung mit größerer Höhe als Breite aufweist, wobei die Öffnung in Richtung ihrer Breite mit einer Hinterschneidung versehen ist, wobei das Einsetzelement einen Stab mit einer am distalen Ende als Verriegelungselement ausgebildeten, sich radial erstreckenden Platte mit größerer Breite als Höhe aufweist, die mittels eines Griffs um die Achse des Stabs verschwenkbar ist.

Grundsätzlich kann die Flexibilität oder Biegsamkeit der Arbeitshülse im distalen Bereich auch in anderer Weise erzielt werden, beispielsweise durch eine in Axialrichtung gegebene Abfolge von jeweils zwei oder drei Umfangsschlitze mit verbleibenden Rippen und Stegen. Die helikal oder schraubenförmige Ausgestaltung hat den Vorteil, dass die Arbeitshülse so leichter durch Schraubbewegung eingeführt werden kann, wozu proximal vorzugsweise ein Radialhebel vorgesehen ist.

Weiterbildungen des Instrumentensets sehen vor, dass das Einsetzinstrument ein Rohr mit einer proximalen Griffhülse aufweist, durch das sich der Stab erstreckt, so dass die Platte relativ zum Rohr verschwenkbar ist vorzugsweise bis zu einen Relativwinkel von 55°, und dass ein Widerlager am Rohr mit zwei parallel zueinander verlaufenden konvexen Stirnkanten ausgebildet ist.

In weiterer bevorzugter Ausgestaltung des erfindungsgemäßen Sets ist vorgesehen, dass Einsetzinstrument und Körbchen jeweils einen durchgängigen Durchlass aufweisen. Hierdurch kann das Einsetzinstrument mit verbundenem Körbchen über einen Führungsdraht in den Zwischenwirbelraum oder das Bandscheibenfach geschoben werden. Weiter kann so das Einsetzinstrument nach Trennen vom im Bandscheibenfach platzierten Körbchen wieder über den Führungsdraht entfernt werden.

Das Körbchen ist vorzugsweise mit netz- oder gitterförmigen Bereichen, insbesondere mit der Struktur einer Diamantgitterzelle ausgebildet. Gitterförmige Bereiche an der Außenseite des Körbchens haben Öffnungsdurchmesser an jeder Öffnung in der Größenordnung von 500 µm bis 700 µm und/oder gitterförmige Bereiche im Inneren des Körbchens haben Öffnungen oder Durchbrüche mit Durchmessern von 1500 µm bis 3200 µm oder einen einheitlichen Öffnungsdurchmesser von 500 µm bis 3200 µm. Die Wandstärke des Materials liegt dabei vorzugsweise in der Größenordnung von 600 µm, insbesondere bei 800 µm.

Das Einsetzen des Körpers geschieht im Wesentlichen derart, dass eine in ihrem distalen Mantelbereich flexibel ausgebildete Arbeitshülse eingeschoben wird, dass nach Entfernen der Bandscheibe aus dem Bandscheibenfach durch die Arbeitshülse ein Körbchen mit zumindest teilweise poröser Struktur mittels eines Einsetzinstruments, mit dem das Körbchen verriegelt ist, in das Bandscheibenfach eingeschoben wird, anschließend die Verriegelung zwischen Körbchen und Einsetzinstrument gelöst und danach die Arbeitshülse entfernt wird. Abgesehen von der Vermeidung der weiteren Nachteile des Standes der Technik ist bei dem Gegenstand der Erfindung insbesondere keine Facettengelenk-Resektion notwendig.

Dabei werden die Instrumente transkutan vom Rücken her unter einem Winkel von mehr als 45° und weniger als 70°, vorzugsweise mehr als 55° und weniger als 65° zum Dornfortsatz bis zum Wirbelkörper eingeführt werden, wobei darüber hinaus die Instrumente vom Rücken her an der der Rückenoberflächen abgewandten Seite eines Querfortsatzes an den Wirbelkörpern eingeführt werden.

Die in ihrem distalen Mantelbereich mit Schlitzen und zwischen diesen angeordneten Stegen versehene Arbeitshülse wird über die Dilatoren eingeführt, wobei diese insbesondere in eine Schraubbewegung über die Dilatoren eingeführt wird. Ein porös ausgebildetes Körbchen wird dann durch die Arbeitshülse in das Bandscheibenfach eingeführt, wobei insbesondere ein Körbchen mit einer im Inneren gröberen und am Außenbereich feineren Porosität eingeführt wird.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt:
- Fig. 1a: eine Seitenansicht eines Wirbelsegments aus zwei Wirbeln;
- Fig. 1b: eine Aufsicht auf einen Wirbel von oben;
- Fig. 1c: eine Aufsicht auf einen Wirbel mit eingebrachtem Körbchen (Cage);
- Fig. 2: Zugangsinstrumentarium bzw. -instrumente der erfindungsgemäßen Vorrichtung;
- Fig. 3a: eine schematische Darstellung eines Körbchens in Form eines O-Cage in perspektivischer Sicht;
- Fig. 3b: eine schematische Seitenansicht des O-Cage der Fig. 3a;
- Fig. 3c: eine Perspektivdarstellung eines Körbchens als P-Cage;
- Fig. 3d: eine Seitenansicht eines P-Cage;
- Fig. 4a: ein Einsetzinstrument und Körbchen (O-Cage) vor der Vorrichtung in Seitenansicht;
- Fig. 4b: einen horizontalen Längsschnitt durch ein Einsetzinstrument in Einführposition;
- Fig. 4c: ein Einsetzinstrument in Verriegelungsposition zum Körbchen, ebenfalls in horizontalem Längsschnitt;
- Fig. 5d: eine vergrößerte Ausschnittsdarstellung von proximalen Teilen des Einsetzinstruments;

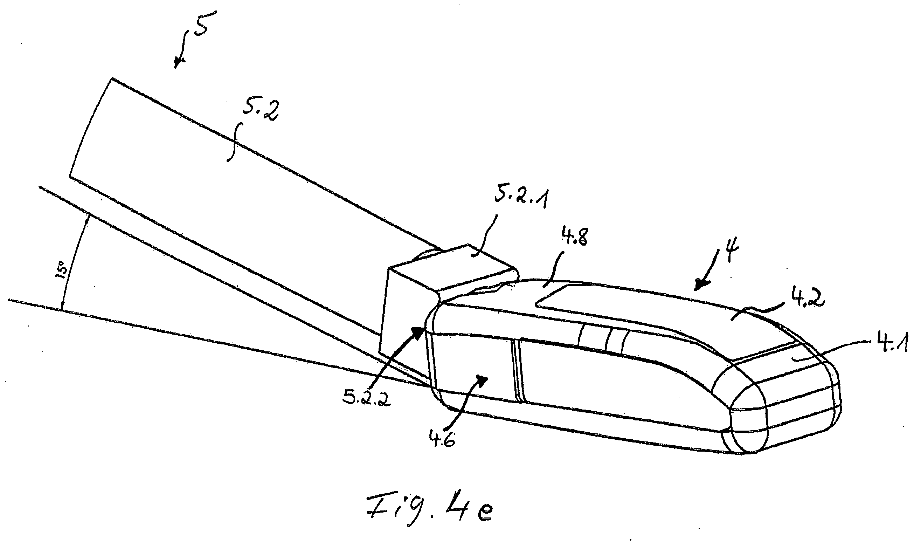

- Fig. 4e: eine Darstellung des distalen Bereichs des Einsatzelements mit angekoppeltem Körbchen zur Verdeutlichung der angularen Beweglichkeit zwischen Einsetzinstrument und Körbchen in vertikaler Richtung bei erfolgter Verriegelung;

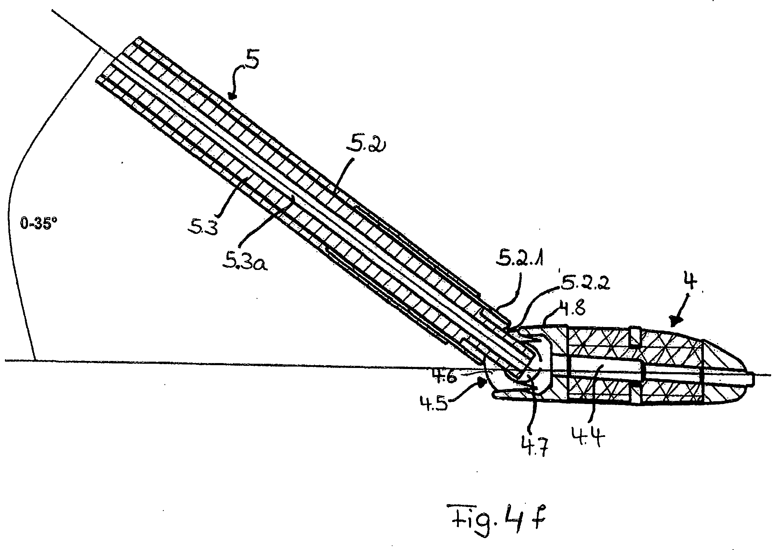

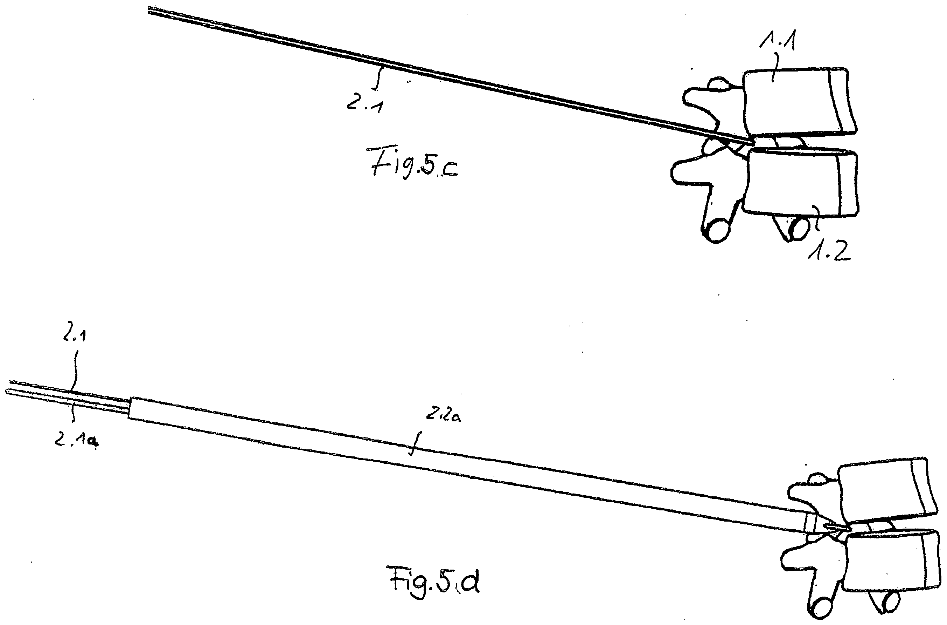

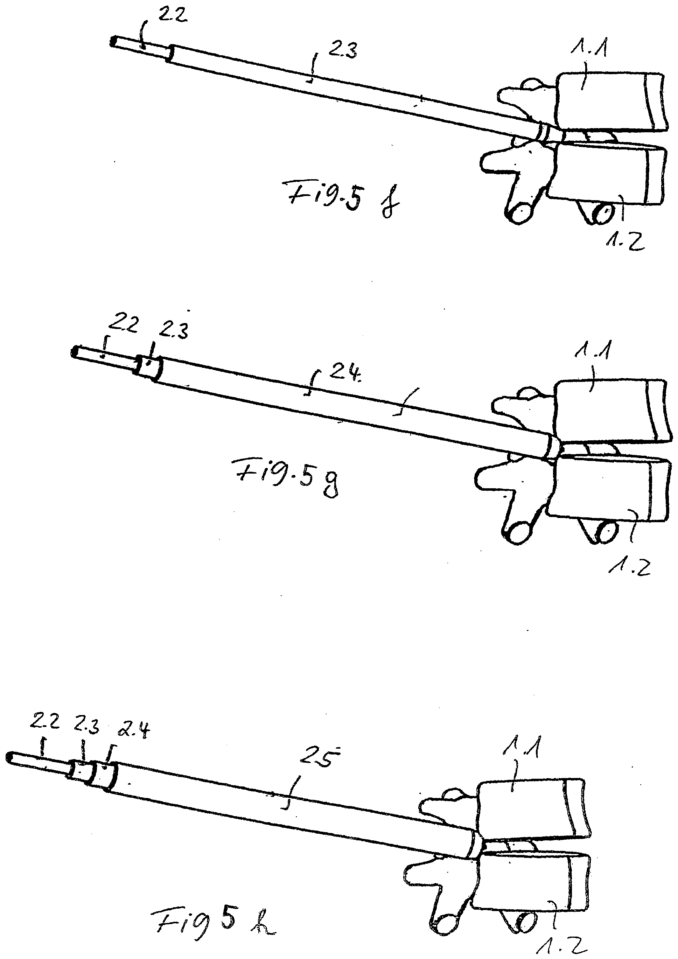

- Fig. 4f: eine Darstellung entsprechend Fig. 5d einer abgewandelten Ausführungsform im Längsschnitt;

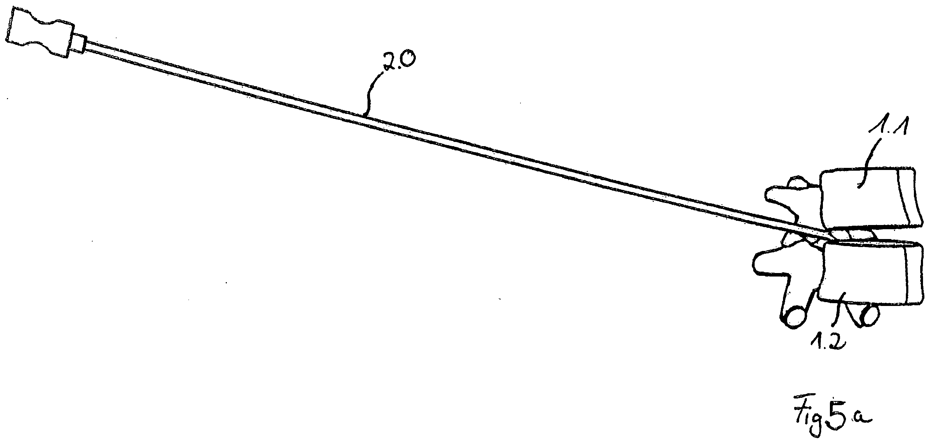

- Fig. 5a: eine durch das Zwischenwirbelloch bis zum Bandscheibenfach platzierte Hohlnadel;

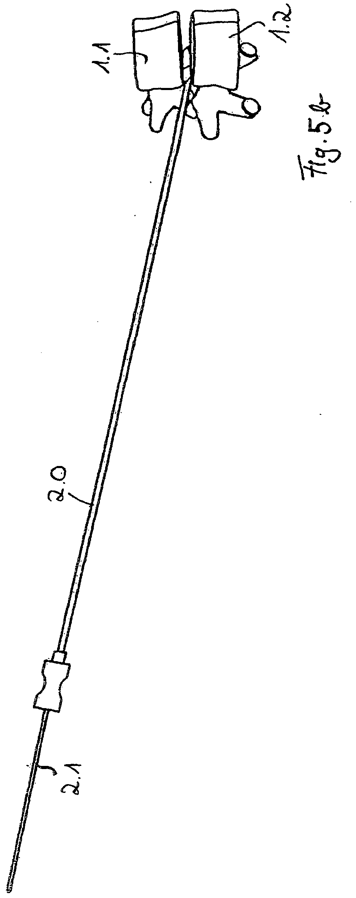

- Fig. 5b: Hohlnadel mit durch diese eingeführter Führungsdraht;
- Fig. 5c: Führungsdraht nach Entfernen der Hohlnadel;
- Fig. 5d: ein über den Führungsdraht eingeführter doppelkanulierter Führungsstab mit weiterem Führungsdraht größerem Durchmessers;

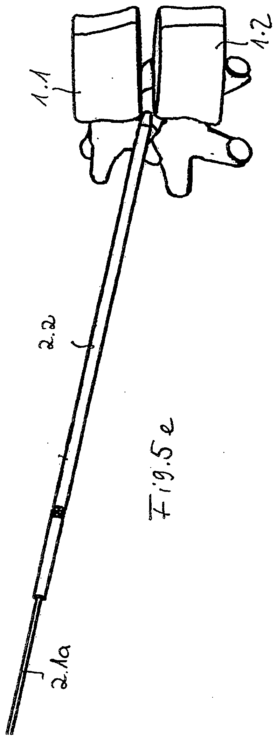

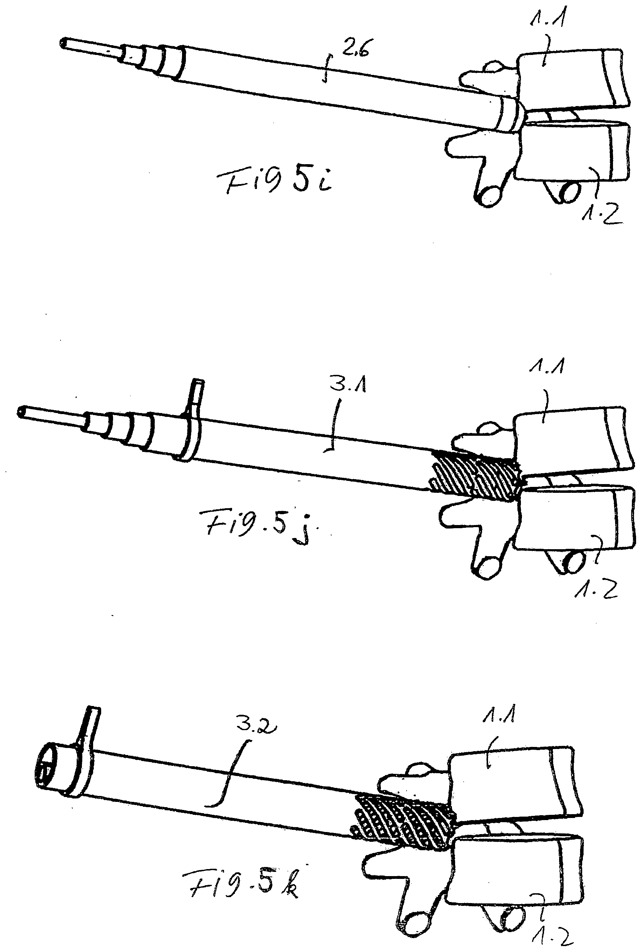

- Fig. 5e -Fig.5i: über den Führungsstab aufgeschobene Dilatoren von ersten Dilator (Führungshülse) (Fig. 5e) bis zum fünften Dilator (Fig. 5i);
- Fig. 5j: über die Dilatoren aufgeschobene Arbeitshülse;
- Fig. 5k: an den Wirbelkörpern der Stirnseite verankerte Arbeitshülse der ersten Ausgestaltung mit flexiblem distalen Endbereich nach Entfernen von Führungsstab und Dilatoren;

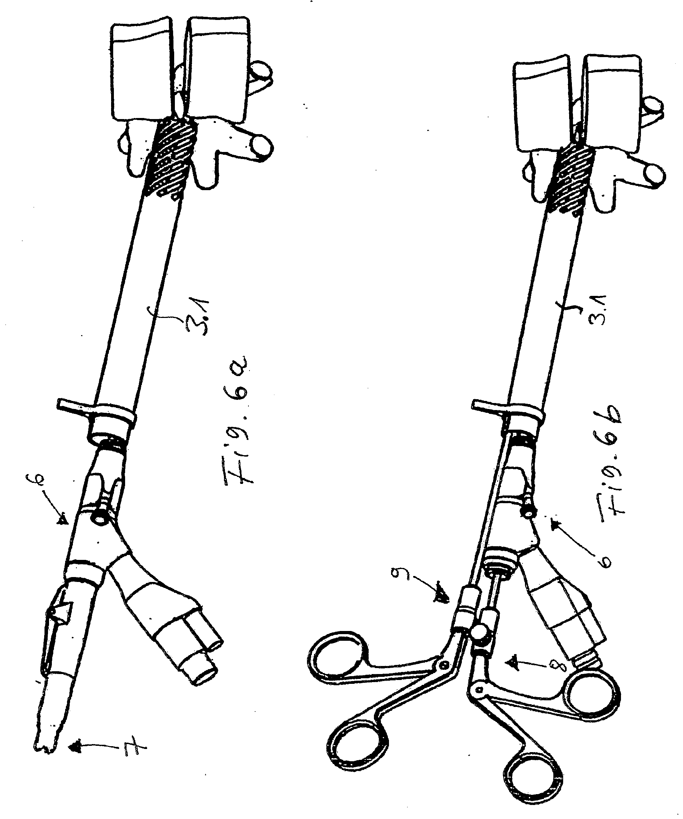

- Fig. 6a: Darstellung eines durch die Arbeitshülse eingeführten Endoskops mit einem durch dieses bis zu den Wirbelkörpern eingeführten drehbaren Fräsers zum Entfernen von Wirbelkörpermaterial zur Erweiterung des Zwischenraums zwischen den Wirbelkörpern;
- Fig. 6b: Darstellung der am Wirbelsegment angedockten Arbeitshülse mit eingeführtem Endoskop sowie durch dieses hindurchgeführten Werkzeugen, in Form zweier verschiedener Zangen zur Entfernung von Bandscheibengewebe; und

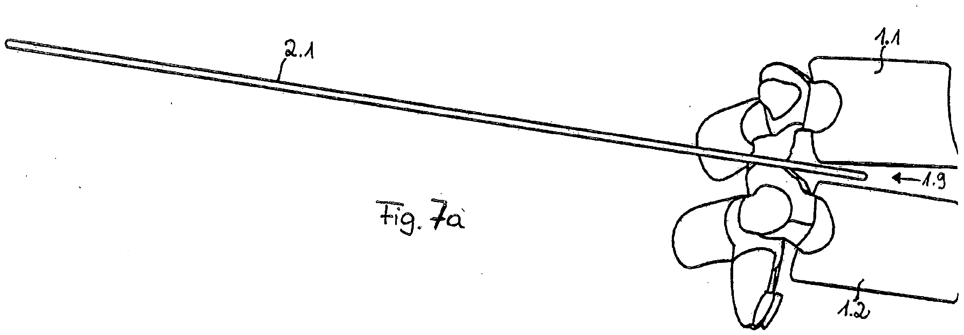

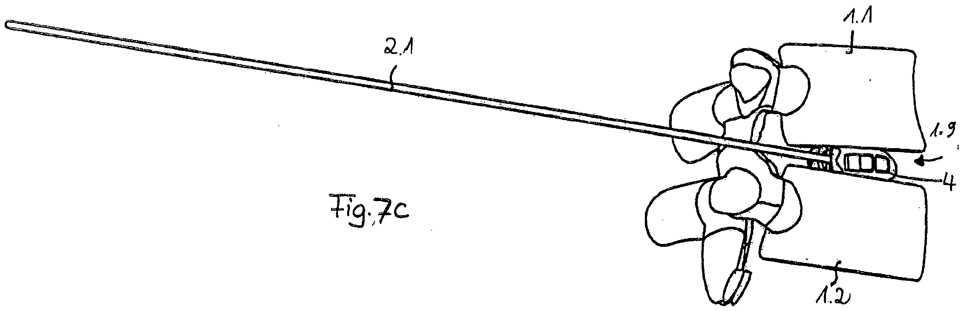

- Fig. 7a - Fig.7c: der Ablauf des Einbringens eines Körbchens mittels eines Einsetzwerkzeugs über einen Führungsdraht in den Wirbelkörperzwischenraum.

Die Erfindung befasst sich mit dem Einbringen eines Körbchens in den üblicherweise von Bandscheibengewebe ausgefüllten Zwischenraum zwischen zwei Wirbelkörpern eines Wirbelsegments, das Bandscheibenfach, zum Ersatz der Bandscheibe und dauerhafter Stabilisierung des Wirbel-segments, üblicherweise als (Lenden-)Wirbelfusion be-zeichnet.

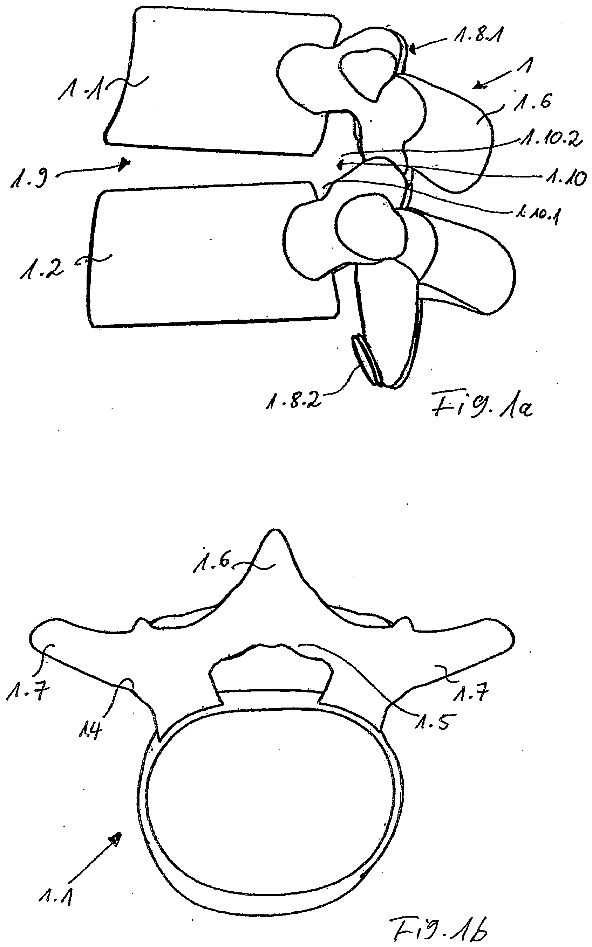

Fig. 1a zeigt ein Wirbelsegment 1 der Wirbelsäule bestehend aus zwei Wirbeln 1.1 und 1.2 und dem Bandscheibenfach 1.9 zwischen diesen, in dem sich die Bandscheibe (nicht dargestellt) befindet, in Seitenansicht. Aus der Aufsicht der Fig. 1b ist erkennbar, dass ein Wirbel 1.1 aus dem Wirbelkörper 1.2, den sich von diesem seitlich des den Spinalkanal bildenden Wirbellochs 1.3 und dieses begrenzend zum Rücken hin erstreckenden Bogenwurzeln 1.4, dem anschließenden Wirbelbogen 1.5 und - von diesem ausgehend und durch diesen verbunden - dem zentralen Dornfortsatz 1.6 sowie den sich seitlich unter Winkeln von etwa 60° erstreckenden Querfortsätzen 1.7 besteht. An den Bogenwurzeln 1.4 sind Wirbelgelenkflächen 1.8.1 und 1.8.2 ausgebildet, wobei eine Wirbelgelenkfläche eines Wirbels mit der des benachbarten Wirbels zusammenwirkt.

Zwischen den beiden Wirbelkörpern 1.1 und 1.2 verbleibt ein Zwischenraum, das Bandscheibenfach 1.9, in dem sich die Bandscheibe (nicht dargestellt) befindet. Zwischen den Bogenwurzeln 1.4 zweier übereinander liegender Wirbel ist ein Zwischenwirbelloch 1.10 oder Foramen Intervertebrale mit begrenzt eine Incisura vertebralis inferior 1.10.1 des oberen Wirbels und einer Incisura vertebralis superior 1.10.2 des unteren Wirbels ausgebildet.

Die Erfindung sieht, wie noch im Folgenden dargestellt wird, den Zugang zum Bandscheibenfach 1.9 zwischen den Wirbel-körpern 1.1 und 1.2 über das Foramen Intervertebrale 1.10 bzw. seitlich daran vorbei vor.

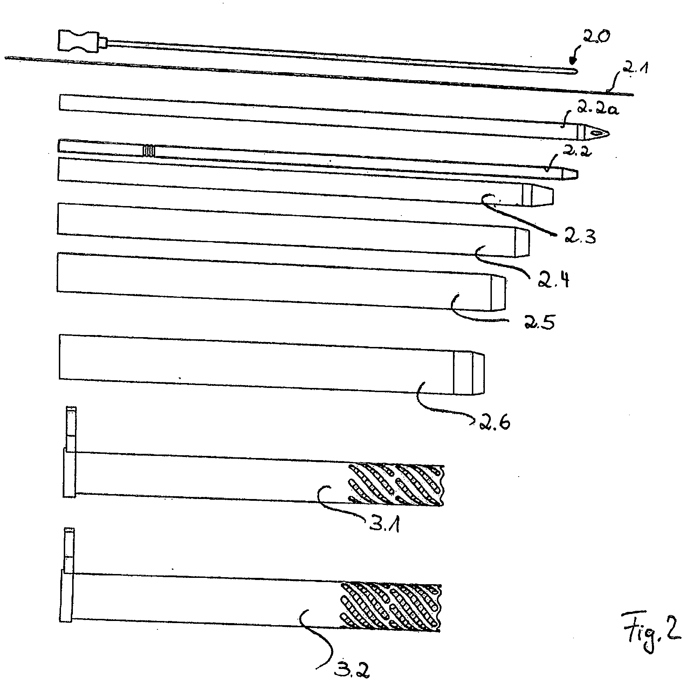

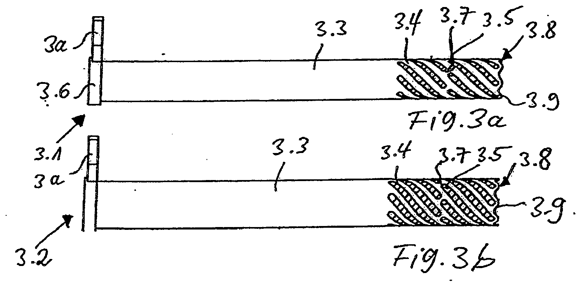

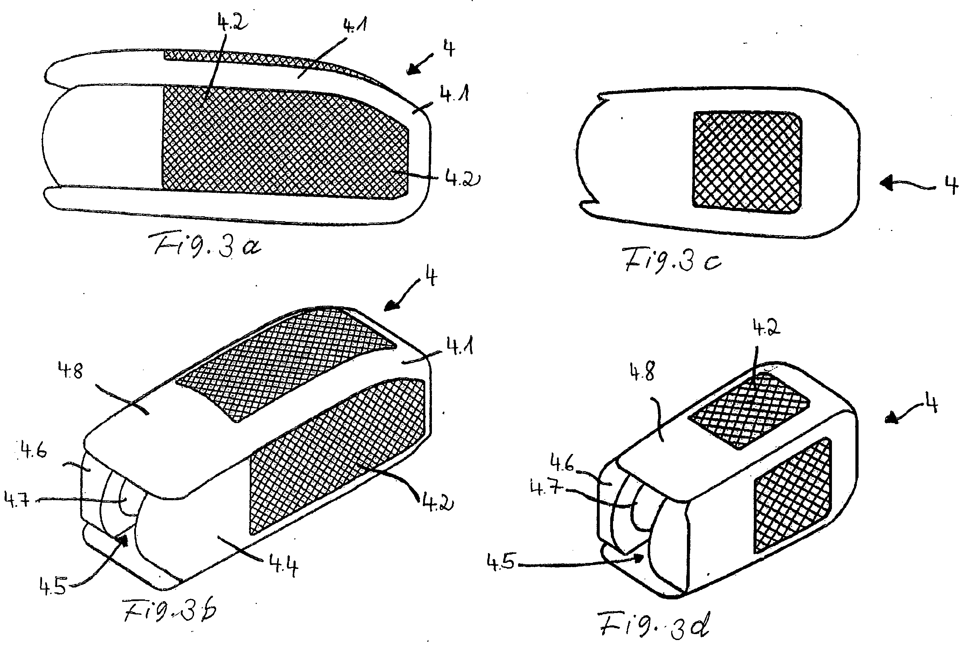

Das erfindungsgemäße Instrumentarium oder Instrumentenset 2 zum Einbringen eines Körbchens (Cage) in das Bandscheibenfach 1.9 weist im dargestellten Ausführungsbeispiel zunächst die folgenden in den Fig. 2 und 3a, 3b dargestellten Instrumente auf: Eine Hohlnadel 2.0, einen Führungsdraht 2.1, der seitlich flexibel, aber axial hinreichend steif ist, um durch insbesondere einen Einschnitt in der Haut eines Patienten unter einem Winkel zwischen 45° und 70°, vorzugsweise zwischen 55° und 65°, seitlich am Zwischenwirbelloch 1.10 vorbei in das Bandscheibenfach 1.9 oder direkt in dieses eingeführt zu werden (Fig. 6a).

Es kann ein doppelkanulierter Führungsstab 2.2a mit zwei Lumina von ca. 1mm und ca. 2mm vorgesehen sein, um gegebenenfalls einen dünneren Führungsdraht 2.1 durch einen stärkeren auszutauschen.

Weiterhin sind ein erster Dilator 2.2 als Führungshülse oder -Stab vorgesehen sowie weitere Dilatatoren oder Dilatoren 2.2-2.6 - im dargestellten Ausführungsbeispiel mit der Führungshülse insgesamt fünf Dilatoren -, wobei je nach Größe des einzusetzenden Körbchens drei, vier oder alle fünf Dilatoren zum Einsatz kommen. Der Innendurchmesser des ersten Dilators 2.2 entspricht dem Außendurchmesser des Führungsdrahts 2.1, der Innendurchmesser des zweiten Dilators 2.3 dem Außendurchmesser des ersten Dilators oder Führungshülse 2.2. Der Innendurchmesser der weiteren Dilatoren 2.4 bis 2.6 entsprechen jeweils dem Außen-durchmesser der Dilatoren 2.3 bis 2.5 geringeren Quer-schnitts. Gegebenenfalls können auch noch mehr Dilatoren vorgesehen sein. Die Dilatoren weisen Außendurchmesser von 8 mm bis 25 mm auf, wobei im dargestellten Ausführungsbeispiel die Dilatoren 2.5 und 2.6 Außendurchmesser von 15 mm bzw. 18 mm aufweisen. Die Wandungsstärke beträgt zwischen 0,5 mm und 2 mm.

Die Dilatoren 2.3 bis 2.6 sind grundsätzlich als Zylindermäntel ausgebildet, aber ihre Wandung ist an ihrem distalen Ende von außen nach innen konisch verjüngt. Außerdem ist die Länge des Dilatators mit jeweils größerem Durchmesser geringer als die Länge des Dilatators mit geringerem Durchmesser, wie dies aus der Fig. 2 ersichtlich ist. Derart können später die Dilatoren von innen her jeweils nacheinander einfach entfernt werden.

Weiter gehört zum Instrumentenset 2 zunächst eine Arbeitshülse 3.1 bzw. 3.2, wie sie in den Fig. 3a bzw. Fig. 3b dargestellt ist, wobei die Arbeitshülse 3.1 der Fig. 3a Durchmesserverhältnisse entsprechend dem Dilatator 2.5 der Fig. 2 hat, so dass sie über den Dilatator 2.4 geschoben werden kann, während der Innendurchmesser der Arbeitshülse 3.2 der Fig. 3b dem Außendurchmesser des Dilatators 2.5 entspricht, so dass erstere über letzteren geschoben werden kann.

Gegebenenfalls können auch drei oder vier Dilatoren ausreichen, über die dann eine Arbeitshülse geringeren Durchmessers geschoben wird.

Bei den Arbeitshülsen 3.1, 3.2 ist der distale Endbereich etwa über ein Viertel bis ein Drittel der Länge der Arbeits-hülse flexibel ausgebildet. Dies ist bei der Ausgestaltung der Fig. 3a, 3b dadurch erreicht, dass im Zylindermantel 3.3 der Arbeitshülse 3.1 (bzw. 3.2) die Wandung vollständig durchsetzende Schlitze 3.4 ausgebildet sind, zwischen denen Rippen 3.5 verbleiben, wobei Rippen 3.5 und Schlitze 3.4 schraubenförmig - von der proximalen Stirnseite 3.6 aus gesehen im Uhrzeigersinn - geführt sind. Die Schlitze 3.4 erstrecken sich nicht durchgehend über den gesamten Umfang, sondern in den dargestellten Ausführungsbeispielen lediglich über etwa einen halben Umfang und sind durch Stege 3.7 unterbrochen, die zwei benachbarte Stege 3.9 verbinden. Darüber hinaus ist auch die distale Stirnseite 3.8 der Zylindermantelwandung der Arbeitshülsen 3.1, 3.2 durch Stege 3.9 geschlossen, so dass die Schlitze nicht frei in die proximale Stirnseite 3.6 auslaufen. Allerdings ist die distale Stirnseite der Mantelwandung 3.3 der Arbeitshülsen 3.1, 3.2 wellenförmig mit Vorsprüngen und Vertiefungen ausgebildet. Hierdurch wird eine sichere Fixierung der Arbeitshülse durch das aufgedehnte Muskelgewebe an der Außenseite der beiden Wirbelkörper 1.1, 1.2 vorgenommen, in deren Bandscheibenfach 1.9 ein Körbchen eingesetzt werden soll (Fig. 1a, 1b). Zum Instrumentenset können weiterhin ein Endoskop sowie Arbeitswerkzeuge, wie Fräser, Raspatorien, Bohrer, Raspeln und Zangen gehören wie sie an sich bekannt sind, aber im Folgenden noch beschrieben werden. Am proximalen Ende 3.6 der Arbeitshülsen 3.1, 3.2 ist ein Radialhebel 3a vorgesehen, mit dem die Arbeitshülsen leicht durch Einschrauben über die Dilatoren eingeführt werden können. Die Arbeitshülsen 3.1, 3.2 werden insbesondere bei O-Cages (O-Körbchen; s.u.) eingesetzt.

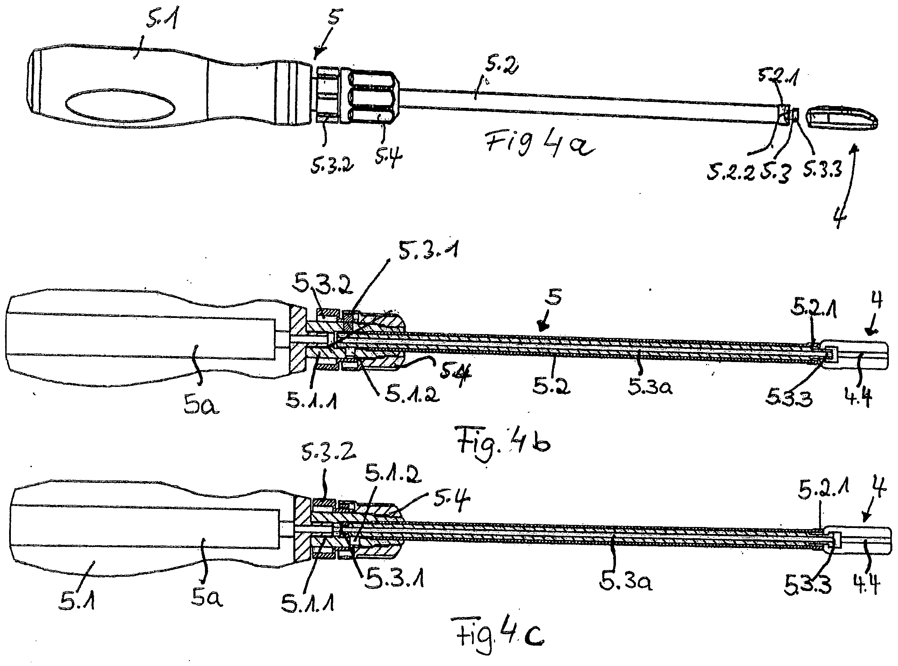

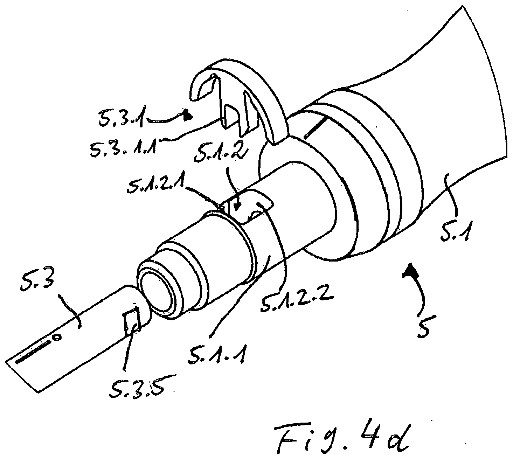

Weiterhin gehört zum erfindungsgemäßen Instrumentenset mindestens ein Körbchen 4 entweder als O- oder Oblique-Cage (Fig. 4a, 4b) oder als P- oder Postorior-Cage (Fig. 4c, 4d - die Qualifizierung der Körbchen bezeichnet die Einführrichtung bzw. den Ansatzort am Körper des Patienten) das jeweils in das Bandscheibenfach 1.9 eingebracht werden soll sowie ein Einsetzinstrument 5 (insbesondere Fig. 5a, 5b), mittels dessen das Körbchen 4 in das Bandscheibenfach 1.9 durch die Arbeitshülse 3.1 bzw. 3.2 eingebracht wird.

Das Körbchen 4 wird durch Elektronenstrahlschmelzen (electron beam melting) aus Titanlegierung, insbesondere TI6AL4V, gemäß ISO 5832-3 hergestellt, wobei das Bauteil durch Aufschmelzen von Metallpulver mittels eines Elektronenstrahls im Hochvakuum hergestellt wird. Hierdurch sind Hinterschneidungen ohne verlorene Formen oder Kerne erzeugbar.

Ein Körbchen 4 kann strukturell drei unterschiedliche Bereiche aufweisen: Zunächst einen massiven Teil 4.1 als tragende Struktur, sodann im Inneren desselben einen Kern mit einer groben Waben- oder Gitterstruktur und am Außenbereich der vier Längsseiten ebenfalls Waben- oder Gitterstruktur 4.2. Die Gitterstruktur der Teile dient dazu, das Einwachsen von Knochenmaterial in das Körbchen 4 zu ermöglichen, um so eine feste Verbindung zwischen Körbchen und benachbarten Wirbelkörpern 1.1, 1.2 zu ermöglichen.

Die Körbchen 4 weisen eine Länge von 22 mm bis 35 mm, vorzugsweise als O-Cages eine Gesamtlänge von 34 mm und eine Breite in der Größenordnung zwischen 10 mm und 15 mm und jeweils eine unterschiedliche Höhe zwischen 6mm bis 16mm je nach Konstitution des Patienten und dem Ort der Einbringung bzw. den Wirbelkörpern und deren Bandscheibenfach, in die das Körbchen eingebracht werden soll von vorzugsweise 8 mm, 10 mm, 12 mm oder 14 mm auf.

Mit diesen Breiten und Höhen sind die Körbchen mit der größten Höhe durch die Arbeitshülse 3.2 und die anderen Körbchen durch die Arbeitshülse 3.1 in das Bandscheibenfach 1.9 zwischen den Wirbelkörpern 1.1 und 1.2 einbringbar. Die Öffnungen der äußeren Waben- oder Gitterstruktur können gleich sein und in der Größenordnung von 0,5 mm bis 3,2 mm liegen. Alternativ kann im Inneren eine gröbere Struktur mit Öffnungen von 1,5 mm bis 3,2 mm und außen eine bessere Struktur mit Öffnungen von 0,5 mm bis 0,7 mm gegeben sein.

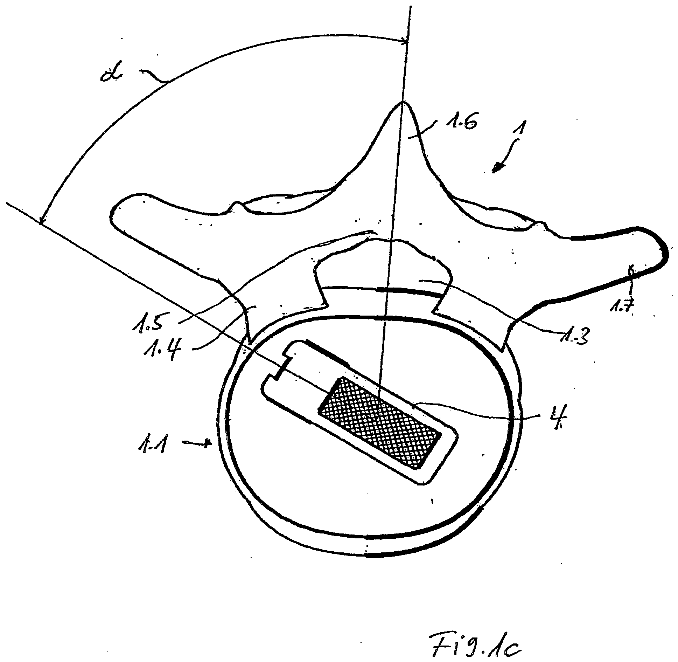

Ein O-Cage (Fig. 4a, 4b) weist eine Länge von größer 30 mm, vorzugsweise von etwa 35 mm auf und wird unter einem Winkel von ca. 60° zum Dornfortsatz 1.6 (Fig. 1c) in das Bandscheibenfach eingeführt (im Einzelnen weiter unten). P-Cages sind kürzer und weisen je nach Patient eine Länge von 24 mm bis 30 mm auf. Sie werden dorsal neben dem Dornfortsatz leicht schräg zu diesem in das Bandscheibenfach eingeführt.

Das Einsetzinstrument 5 weist zunächst einen Griff 5.1 und ein (erstes) äußeres Rohr 5.2 auf, das über ein Zwischenteil 5.1.1 fest mit dem Griff 5.1 verbunden ist. Durch das äußere Rohr 5.2 erstreckt sich ein Stab oder - hier - ein (zweites) inneres Rohr 5.3.

Der Griff 5 weist einen axialen Durchbruch 5a auf, der mit dem Hohlraum 5.3a des inneren Rohres 5.3 fluchtet. Der innere Hohlraum 5.3a mündet in einer distalen Öffnung 5.3b. Derart ist vom proxialen Ende des Durchbruchs 5.1a bis zum distalen Ende des inneren Rohrs 5.3 ein durchgehender Durch-lass 4.4 gebildet (Fig. 5f).

Hierdurch kann ein Körbchen 4 das ebenfalls einen durchgehenden Durchlass aufweist mit dem Einsetzinstrument 5 über einen liegenden Führungsdraht durch diesen geführt in den Zwischenwirbelraum (das Bandscheibenfach) eingebracht werden.

Mit dem proximalen Ende des inneren Rohrs 5.3 ist über einen Verriegelungsbügel 5.3.1 ein Arretierrad 5.3.2 verbunden. Der Verriegelungsbügel 5.3.1 ist am Umfang des inneren Rohres 5.3 fest mit diesem verbunden. Der Verriegelungsbügel 5.3.1 erstreckt sich durch einen Radialdurchbruch 5.1.2 des Zwischenteils 5.1.1, der ein Verschwenken des Stabs 5.3 über 90° relativ zum Zwischenteil 5.1.1 und damit auch zu Griff 5.1 und Rohr 5.2 erlaubt. Hierzu erstreckt sich der Durchbruch über ca. 90° in angularer Richtung.

An einer angularen Endposition - und zwar in der Verriegelungsposition des distalen Verriegelungselements mit Querriegel 5.3.3 (Fig. 5c) - erstreckt sich der Durchbruch 5.1.2 achsparallel in proximaler Richtung, so dass in diesen Positionen das innere Rohr 5.3 zusammen mit Arretierrad 5.3.2 relativ zu Griff 5.1 und Rohr 5.2 in proximaler Richtung verschiebbar ist. Weiter ist unmittelbar distal des Arretierrads 5.3.2 ein Verriegelungsrad 5.4 auf dem Zwischenteil 5.2.1 angeordnet, das mit dem Zwischenteil 5.1.2 über eine Gewindeverbindung 5.4.1 verbunden ist.

Die proximalen Arretierteile sind näher in der Fig. 5d dargestellt. Ihre Anordnung am Einsetzelement ist in Verbindung mit den Fig. 5b und 5c zu sehen.

Die Fig. 5d zeigt ein vergrößert mit dem Griff 5.1 verbundenes Zwischenteil 5.1.1 und in diesem den - einseitigen - radialen Durchbruch 5.1.2 mit seinem sich angular erstreckenden Abschnitt 5.1.2.1 und einen sich axial erstreckenden Abschnitt 5.1.2.2. Am proximalen Ende des inneren Rohrs 5.3 sind seitlich zwei sich diagonal gegenüberliegende Abflachungen 5.3.5 vorhanden. An diesen greift axial- und drehfest der Verriegelungsbügel mit zwei sich parallel radial durch den Durchbruch 5.1.2 erstreckenden Zapfen 5.3.1.1 an. Die Zapfen können sich dabei angular in den Abschnitt 5.1.2.1 bzw. axial in den Abschnitt 5.1.2.2 des Durchbruchs bewegen. Das angulare Bewegen erfolgt in der Lösestellung des Verriegelungsrads 5.4 durch seitliches Angreifen des Arretierrads 5.3.2 (Fig. 5b, Fig. 5c), während das axiale Bewegen des Verriegelungsbügels über den äußeren Bügelbogen 5.2.1.2 desselben durch das Verriegelungsrad 5.4 selbst erfolgt (Fig. 5c), wie vorstehend beschrieben.

Am distalen Ende des inneren Rohrs 5.3 ist dieses mit einem hammerartigen Verriegelungselement versehen, das ein sich quer erstreckender distale Querriegel 5.3.3 mit unterschiedlicher Höhe und Breite aufweist - jeweils senkrecht zur Erstreckung des inneren Rohres 5.3. Der Querriegel 5.3.3 hat hierzu das innere Rohr 5.3 radial überragende Radial-ansätze 5.3.4. Am distalen Ende des äußeren Rohrs 5.2 ist ein Widerlager 5.2.1 mit parallel konkav ausgebildeten (vertikalen) Stirnkanten 5.2.2 angeordnet.

Ein Körbchen 4 weist an einer Stirnseite eine hinterchnittene Öffnung 4.5 auf, deren Öffnungsquerschnitt dem der Querriegel 5.3.3 des Einsetzinstruments 5 entspricht. Die Hinterschneidung wird dadurch gebildet, dass auf den Innen-seiten im proximalen Bereich der Seitenwände 4.6 kreis-förmige Vertiefungen 4.7 ausgebildet sind. In diese greifen die Queransätze 5.3.4 in der Verriegelungsstellung des Ver-riegelungsinstruments 5 mit dem Körbchen 4 ein. Die hinter-schnittene Öffnung 4.5 bildet ein Verriegelungselement am Körbchen 4, wodurch eine Verriegelung von Einsetzelement 5 und Körbchen 4 ermöglicht wird.

Zum Verbinden des Einsetzinstruments 5 mit einem Körbchen 4 wird das Verriegelungsrad 5.4 auf dem Zwischenteil 5.1.1 über die Gewindeverbindung 5.4.1 in distaler Richtung verdreht (bei üblichem Gewinde und Blick vom Griff 5.1 im Uhrzeigersinn) Hierdurch wird das Arretierrad 5.3.2 freigegeben und mit ihm der Verriegelungsbügel 5.3.1, so dass dieser aus einem der achsparallelen Endbereiche des Durchbruchs 5.1.2 in dessen angularen Bereich gelangen kann.

Hierdurch kann der Querriegel 5.3.3 des Einsetzinstruments 5 bei vertikaler Ausrichtung (Fig. 5a, 5b) in die Öffnung 4.1 des Körbchens 4 eingeführt werden, wobei die Erstreckungs-richtungen größter Abmessung von Querriegel 5.3.3 und Öffnung 4.5 übereinstimmen.. Nach Einführen des Querriegels 5.3.3 durch die Öffnung 4.5 des Körbchens wird der Quer-riegel 5.3.3 um 90° verschwenkt. Hierzu wird das Arretierrad 5.3.2 über den Verriegelungsbügel 5.3.1 und mit diesem der Stab 5.3 und die Platte 5.3.3 um 90°relativ zum Griff 5.1 in eine Arretierposition des Querriegels 5.3.3 verschwenkt, so dass dieser in die Vertiefungen 4.5 eingreift und damit die Hinterschneidungen hinter der Öffnung 4.5 hintergreift (Fig. 5a). Anschließend erfolgt ein Verschrauben des Ver-riegelungsrads 5.4 (im Gegenuhrzeigersinn) über die Schraub-verbindung 5.4.1 relativ zum Griff 5.1. Dadurch wird der Verriegelungsbügel 5.3.1 proximal in einen der achs-parallelen Bereiche des Durchbruchs 5.2.1 gedrückt und damit der Querriegel 5.3.3 gegen den die Hinterschneidung bildenden proximalen Stirnwandbereich des Körbchens 4 und dieses gegen das Widerlager 5.2.1 des Einsetzinstruments 5 gezogen und damit verspannt. Die Stärke der Verspannung kann dabei in gewünschter Weise gewählt werden. Insbesondere aufgrund der konkaven Ausbildung der Stirnseiten des Wider-lagers 5.2.1 kann eine Relativerschwenkung von Körbchen 4 und Einsetzteil 5 um bis zu ca. 15° erfolgen, wie dies in der Fig. 5d dargestellt ist. Durch eine Erstreckung der Einführöffnung 4.5 bis in den Bereich der oberen Wandung 4.8 des Körbchens 4 kann der Verschwenkwinkel noch vergrößert werden, vorzugsweise bis auf ca. 35° (Fig. 5e). Das Lösen von Einsetzelement 5 und Körbchen 4 erfolgt in ent-sprechender Weise; die vorgenannten Schritte werden im Wesentlichen in umgekehrter Reihenfolge durchgeführt. Einsetzinstrument 5 und ein gewähltes Körbchen 4 werden in der oben unter Bezug auf die Fig. 5a bis 5d beschriebenen Weise miteinander verbunden. Das Körbchen 4 wird dann verbunden mit dem Einsetzinstrument 5 durch die Arbeitshülse 3.1, 3.2 - vorzugsweise unter Röntgenkontrolle - in das Bandscheibenfach 1.9 eingeführt.

Ein Körbchen 4 wird nach dem folgenden Verfahren in das Bandscheibenfach 1.9 eingeführt:
Der Patient befindet sich vorzugsweise in stabiler Seitenlage (auch Bauchlage ist möglich) und ist durch eine Analogsedierung während der Operation ständig ansprechbar (er benötigt also keine Vollnarkose, wobei diese auch möglich ist). Der Operateur nimmt einen Einschnitt in der Haut des Rückens seitlich der Wirbelsäule (ca. 8-18 cm neben dem Dornfortsatz) vor, durch den dann unter einem Winkel zwischen 55° und 65°, vorzugsweise von ca. 60°, zuerst eine Hohlnadel 2.0 direkt entlang der dem Rücken abgewandten Seite eines Querfortsatzes in das Bandscheibenfach 1.9 vom Operateur (Fig. 6a) platziert wird. Der Spinalkanal 1.3, durch welchen die Nerven geführt sind, wird dabei nicht tangiert.

Durch diese Nadel 2.0 wird ein Führungsdraht 2.1 eingeführt (Fig. 6b). Die Nadel 2.0 wird entnommen und der Führungsdraht bleibt stehen (Fig. 6c). Über den Führungsdraht 2.1 wird ein doppelkanulierter Führungsstab 2.2a mit zwei exzentrischen Bohrungen eingeführt (Fig. 6d). Mit Hilfe dieses Führungsstabes 2.2a kann ein anderer Draht 2.1a mit höherer Steifigkeit und gegebenenfalls größerem Durchmesser eingebracht werden. Der Führungsstab 2.2a und der erste Führungsdraht 2.1 werden entnommen, und der zweite Führungs-draht 2.1a bleibt stehen. Anschließend wird über den (zweiten) Führungsdraht 2.1a der erste Dilator 2.2 durch das Foramen Intervertebrale bis zum Bandscheibenfach 1.9 geschoben. Sodann wird der - ggf. zweite - Führungsdraht 2.1 bzw. 2.1a entfernt und der erste Dilator 2.2 dient als Führungshülse für die weiteren Dilatoren 2.3 bis 2.5 (bzw. 2.6). In der Folge werden sukzessive die weiteren Dilatoren 2.3, 2.4, 2.5 und eventuell 2.6 jeweils über den vorher eingeführten Dilator geschoben (Fig. 6e bis 6f).

Anschließend wird über den letzten Dilator (entweder 2.5 oder 2.6) eine der Arbeitshülsen 3, 3.2 bis zur Außenseite der Wirbelkörper 1.1, 1.2 eingeschoben und dort mit der distalen wellenförmigen Stirnseite 3.6 verankert (Fig. 6g). Sodann werden die Dilatoren 2.2-2.5 (bzw. auch 2.6) aus der Arbeitshülse 3 bzw. 3.2 wieder in proximaler Richtung herausgezogen, so dass nur die Arbeitshülse verbleibt (Fig. 6h). In gleicher Weise kann grundsätzlich auch eine Arbeitshülse 3a der zweiten Ausgestaltung (Keilhülse) über Dilatoren 2.2 bis 2.5 (2.6) eingeführt werden, deren Endposition in Fig. 6i dargestellt ist. Dabei greifen die Vorsprünge 3a.1 zwischen den benachbarten Wirbeln in das Bandscheibenfach und halten die Wirbel dabei auf Abstand.

Durch die Arbeitshülse kann nun ein Endoskop 6 eingeführt werden (Fig. 7a) und durch dieses unter endoskopischer Sicht geeignete Werkzeuge, wie zunächst ein Fräser 7. Mittels des Fräsers kann durch die Arbeitshülse 3 hindurch Bandscheiben-material und Knochengewebe am Umfang der Wirbelkörper 1.1, 1.2 entfernt werden. Mit Fräsern, Bohrern, Raspatorien, Zangen oder durch Shaver Blades wird das Bandscheibenfach 1.9 für die Körbchen 4 (O-Cage oder P-Cage) passgenau platziert.

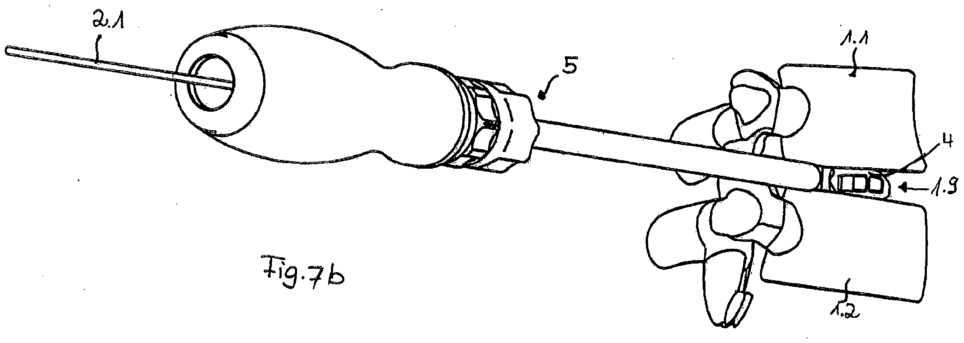

Anschließend wird der Fräser 7 aus dem Endoskop 6 wieder entfernt und durch dieses können weitere Instrumente, insbesondere Zangen 8, 9 eingeführt werden, um Material der Bandscheibe aus dem Bandscheibenfach 1.9 zu entfernen Fig. 7b).

Die Wirbelkörper können dabei in geeigneter Weise auf Abstand gehalten werden, wie durch Pedikelschrauben, Keilhülsen oder dergleichen (was nicht Gegenstand der vorliegenden Erfindung ist).

Nach Entfernen des Bandscheibenmaterials aus dem Bandscheibenfach 1.9 wird das Endoskop 6 und werden die Werkzeuge 8, 9 aus der Arbeitshülse 3 entfernt. Anschließend wird mittels des Einsetzinstruments 5 durch die Arbeitshülse 3 das Körbchen 4 in das Bandscheibenfach eingeführt, wie dies in den Figuren 8a - 8c dargestellt ist. Das Einbringen des Körbchens 4 in das Bandscheibenfach 1.9 erfolgt unter Röntgensicht, wobei das Material des Körbchens 4 (Titanlegierung in Wabenstruktur) eine gute Beobachtung des Einbringens ermöglicht.

Bei der Darstellung des Einbringens eines Körbchens 4 in das Bandscheibenfach 1.a (Wirbelzwischenraum) in den Fig. 8a-8c wurde der besseren Übersicht wegen die weiterhin entsprechend der Fig. 6h oder 6d verbundenen Arbeitshülse nicht wiedergegeben.

Soweit der Führungsdraht 2.1 oder 2.1a entfernt wurde und beim Entfernen der Bandscheibe mittels durch das Endoskop 6 arbeitenden Werkzeuge (Fig. 7a,b) nicht liegengeblieben ist, wird vor Entfernen des Endoskops 6 durch dieses erneut ein Führungsdraht 2.1 oder 2.1a eingeführt und dieses dann entfernt. Daraufhin befinden sich im Körper des Patienten gemäß Fig. 8a ein Führungsdraht, beispielsweise Führungsdraht 2.1 und die Arbeitshülse (aus den oben genannten Gründen nicht wiedergegeben). Anschließend wird ein mit einem Einsetzwerkzeug 5 in der oben beschriebenen Weise verbundenen Körbchen 4 mittels des Einsetzinstruments 5 über den Führungsdraht 2.1 und durch die Arbeitshülse in das Bandscheibenfach 1.9 eingeschoben (Fig. 8b).

Nach Einbringen des Körbchens 4 mittels des Einsetzinstruments 5 in das Bandscheibenfach 1.9 und geeigneter Positionierung in diesem erfolgt ein Lösen der Verriegelung mittels des Verriegelungsrades 5.4 und ein Rückverdrehen der Platte 5.3.3 mittels des Arretierrades 5.3.2 (Fig. 5b, 5c), wodurch das Einsetzelement 5 vom Körbchen 4 getrennt und das Einsetzelement 5 durch die Arbeitshülse 3 wieder in proximaler Richtung aus der Arbeitshülse 3 oder dem Führungsdraht 2.1 entfernt werden kann.

Mit dem Endoskop 6 wird anschließend das Körbchen 4 in der Position auch direkt kontrolliert und inspiziert. Unter endoskopischer Sichtkontrolle kann auch das Körbchen 4 durch die sichtbare Vertiefung (Hinterschnitt) zusätzlich mit Knochen (Knochenersatzstoff) befüllt werden. Schließlich werden noch der Führungsdraht 2.1 und die Arbeitshülse 3 entfernt, während das Körbchen 4 im Bandscheibenfach 1.9 verbleibt. Anschließend wird die Arbeitshülse 3 entfernt, die Wunde verschlossen und versorgt, womit die Operation beendet ist.

Im Rahmen des chirurgischen Eingriffs können die beiden im Bandscheibenfach begrenzenden Wirbel noch mittels eines Schrauben-Stab-Systems, Peditralschrauben od. dgl. verspannt werden. Dies ist nicht Gegenstand der Erfindung und daher nicht dargestellt und nicht beschrieben.

## Patentansprüche

1. Instrumentenset zum Einbringen eines Körbchens in das Bandscheibenfach zwischen zwei Wirbelkörpern, mit einem Führungsdraht (2.1), mit mehreren über den Führungsdraht und übereinander aufschiebbaren Dilatoren (2.3-2.6), mit einer Arbeitshülse (3) und einem Körbchen (4), **dadurch gekennzeichnet, dass** die Arbeitshülse (3) in ihrem distalen Mantelbereich mit Schlitzen (3.4) mit dazwischen angeordneten Rippen (3.5) versehen ist, die ein Festlegen des distalen Bereichs der Arbeitshülse (3) in Richtung ihrer Erstreckung bei variabler angularer Ausrichtbarkeit ihres proximalen Endes ermöglichen.

2. Set nach Anspruch 1, **dadurch gekennzeichnet, dass** die Schlitze (3.4) um den Mantel (3.3) der Arbeitshülse (3) schraubenlinig verlaufen, wobei insbesondere auf einer Schraubenlinie mindestens zwei Schlitze durch einen Steg (3.6) getrennt aufeinander fluchtend angeordnet sind.

3. Set nach einem der Ansprüche 1 oder 2, **dadurch gekennzeichnet, dass** die Breiten von Schlitzen (3.4), Rippen (3.5) und Stegen (3.6) zwischen 0,5 mm und 2 mm, vorzugsweise zwischen 0,8 und 1,2 mm, liegen.

4. Set nach einem der vorangehenden Ansprüche, **dadurch gekennzeichnet, dass** das Körbchen (4) und ein Einsetzinstrument (5) zum Einbringen des Körbchens (4) jeweils miteinander zusammenwirkende Verriegelungselemente (4.1, 4.2; 5.5, 5.7) aufweisen, die eine gemeinsame Verriegelung bilden.

5. Set nach Anspruch 4, **dadurch gekennzeichnet, dass** die Verriegelung von Einsetzinstrument (5) und Körbchen (4) eine angulare Beweglichkeit beider Teile (5, 4) erlaubt.

6. Set nach einem der vorangehenden Ansprüche, **dadurch gekennzeichnet, dass** das Körbchen (4) an einer Stirnseite als Verriegelungselement eine Öffnung (4.1) mit größerer Höhe als Breite aufweist, wobei die Öffnung (4.1) in Richtung ihrer Breite mit einer Hinterschneidung (4.2) versehen ist.

7. Set nach einem der vorangehenden Ansprüche, **dadurch gekennzeichnet, dass** ein Einsetzelement (5) einen Stab oder ein inneres Rohr (5.3) mit einer am distalen Ende als Verriegelungselement ausgebildeten, sich radial erstreckenden Platte mit größerer Breite als Höhe aufweist, die mittels eines Griffs (5.4) um die Achse des Stabs (5.3) verschwenkbar ist.

8. Set nach Anspruch 7, **dadurch gekennzeichnet, dass** das Einsetzinstrument (5) ein äußeres Rohr (5.1) aufweist, durch das sich der Stab (5.3) erstreckt, so dass die Platte (5.5) relativ zum Rohr (5.1) verschwenkbar ist und durch die Griffhülse (5.2) arretierbar ist.

9. Set nach Anspruch 7, **gekennzeichnet durch** ein Widerlager (5.6) am Rohr (5.1) mit zwei parallel zueinander verlaufenden konvexen Stirnkanten (5.7), wobei insbesondere Einsetzinstrument (5) und Körbchen (4) jeweils einen durchgängigen Durchlass aufweisen.

10. Set nach einem der vorangehenden Ansprüche, **dadurch gekennzeichnet, dass** das Körbchen (4) mit netz- oder gitterförmigen Bereichen (4.2, 4.3) ausgebildet ist.

11. Set nach Anspruch 10, **dadurch gekennzeichnet, dass** die gitterförmigen Bereiche (4.3) Öffnungsdurchmesser jeder Öffnung von 0,5 mm bis 3,2 mm aufweisen, dabei vorzugsweise an der Außenseite des Körbchens Öffnungsdurchmesser jeder Öffnung in der Größenordnung von 0,5 mm bis 0,7 mm und/oder gitterförmige Bereiche im Inneren des Körbchens mit Öffnungen oder Durchbrüchen mit Durchmessern von 1,5 mm bis 3,2 mm aufweisen.

## Claims

1. Instrument set for inserting a cage into the intervertebral disc space between two vertebral bodies, with a guide wire (2.1), with several over the guide wire and one over another deferrable dilators (2.3 - 2.6), with a working sleeve (3) and with a cage (4), **characterized in that** the working sleeve is in its distal jacket area equipped with slots (3.4) including in between arranged ribs (3.5), which do enable a fixation of the distal area aof the working sleeve (3) in direction of its extension while the proximal end has variable angular mobility.

2. Set in accordance with claim 1, **characterized in that** the slots (3.4) around the jacket (3.3) of the working sleeve (3) do extend in helical line, whereupon in particular on a helical curve at least two slits are separated through a web (3.6) and arranged flush on top of each other.

3. Set in accordance with claim 1 or 2, **characterized in that** the widths of the slots (3.4), ribs (3.5) and webs (3.6) do range between 0,5 mm and 2 mm, pereferably between 0,8 mm and 1,2 mm.

4. Set in accordance with one of the preceeding claims, **characterized in that** the cage (4) and an inserting instrument (5) for inserting the cage (4) do respectively exhibit locking elements (4.1, 4.2; 5.5, 5.7) with each other interacting, which do form a common locking.

5. Set in accordance with claim 4, **characterized in that** the locking of the inserting instrument (5) and the cage (4) does permit an angular flexibility of both components (5, 4) .

6. Set in accordance with one of the preceeding claims, **characterized in that** the cage (4) does exhibit an opening (4.1) with a height that is greater than the width as a locking element on a front side, wherein the opening (4.1) is equiped with an undercut (4.2) in the direction of its width.

7. Set in accordance with one of the preceeding claims, **characterized in that** the inserting instrument (5) has a rod or an inner tube (5.3) with a radially extending plate, which is designed as a locking element at the distal end, extends radially and has a greater width than height, and which is pivotable along the axis of the rod (5.3) by means of a grip (5.4).

8. Set in accordance with claim 7, **characterized in that** the inserting instrument (5) has an outer tube (5.1), through which the rod (5.3) does extend, so that the plate (5.5) is pivotable relative to the tube (5.1) and by which the grip sleeve (5.2) is lockable.

9. Set in accordance with claim 7, **characterized by** an abutment (5.6) at the tube (5.1) with two convex front edges (5.7) extending in parallel to one another, whereby in particlar the inserting instrument (5) and the cage (4) have a through passage each.

10. Set in accordance with one of the preceeding claims, **characterized in that** the cage (4) is designed with gridlike or lattice-like areas (4.2, 4.3).

11. Set in accordance with claim 10, **characterized in that** the lattice-like areas (4.3) have opening diameters with each opening ranging from 0.5 mm to 3.2 mm, and each opening preferably has opening diameters in the order of magnitude from 0.5 mm to 0.7 mm on the outer side oft he cage and/or lattice-like areas in the interior of the cage have openings or perforations with diameter ranging from 1.5 mm to 3.2 mm.

## Revendications

1. Ensemble d'instruments pour introduire une cage dans un espace intervertébral entre deux corps vertébraux, avec un fil de guidage (2.1), avec plusieurs dilatateurs (2.3-2.6) pouvant être poussés via le fil de guidage et les uns au-dessus des autres, avec un manchon de travail (3) et une cage (4), **caractérisé en ce que** le manchon de travail (3) est pourvu dans sa zone d'enveloppe distale de fentes (3.4) avec des nervures (3.5) disposées entre et permettant de fixer la zone distale du manchon de travail (3) en direction de leur extension en faisant varier l'orientation angulaire de leur extrémité proximale.

2. Ensemble selon la revendication 1, **caractérisé en ce que** les fentes (3.4) s'étendent en forme d'hélice autour de l'enveloppe (3.3) du manchon de travail (3), notamment au moins deux fentes sur une hélice étant disposées de façon à être alignées l'une par rapport à l'autre tout en étant séparées par un étai (3.6).

3. Ensemble selon l'une quelconque des revendications 1 ou 2, **caractérisé en ce que** les largeurs des fentes (3.4), des nervures (3.5) et des étais (3.6) sont comprises entre 0,5 mm et 2 mm, de préférence entre 0,8 et 1,2 mm.

4. Ensemble selon l'une quelconque des revendications précédentes, **caractérisé en ce que** la cage (4) et un instrument d'insertion (5) servant à introduire la cage (4) comportent des éléments de verrouillage (4.1, 4.2; 5.5, 5.7) interagissant respectivement entre eux et formant un verrouillage commun.

5. Ensemble selon la revendication 4, **caractérisé en ce que** le verrouillage de l'instrument d'insertion (5) et de la cage (4) permet une mobilité angulaire des deux pièces (5, 4).

6. Ensemble selon l'une quelconque des revendications précédentes, **caractérisé en ce que** la cage (4) comporte une ouverture (4.1) servant d'élément de verrouillage avec une hauteur plus importante que la largeur au niveau d'un côté avant, l'ouverture (4.1) étant pourvue d'un détouré arrière (4.2) en direction de sa largeur.

7. Ensemble selon l'une quelconque des revendications précédentes, **caractérisé en ce qu'**un élément d'insertion (5) comporte une tige ou un tube intérieur (5.3) avec une plaque réalisée sous la forme d'un élément de verrouillage au niveau de l'extrémité distale et s'étendant dans le plan radial, avec une largeur plus importante que la hauteur et pouvant être pivotée autour de l'axe de la tige (5.3) à l'aide d'une poignée (5.4).

8. Ensemble selon la revendication 7, **caractérisé en ce que** l'instrument d'insertion (5) comporte un tube extérieur (5.1) à travers lequel la tige (5.3) s'étend, de sorte que la plaque (5.5) puisse être pivotée par rapport au tube (5.1) et puisse être arrêtée par le manchon de préhension (5.2).

9. Ensemble selon la revendication 7, **caractérisé par** la présence d'un contre-palier (5.6) prévu au niveau du tube (5.1) avec deux arêtes avant (5.7) convexes s'étendant parallèlement l'une par rapport à l'autre, notamment l'instrument d'insertion (5) et la cage (4) comportant respectivement un passage traversant.

10. Ensemble selon l'une quelconque des revendications précédentes, **caractérisé en ce que** la cage (4) est réalisée avec des zones (4.2, 4.3) en forme de filet ou de treillis.

11. Ensemble selon la revendication 10, **caractérisé en ce que** les zones (4.3) en forme de treillis présentent des diamètres d'ouverture pour chaque ouverture de 0,5 mm à 3,2 mm, en l'occurrence de préférence au niveau du côté extérieur de la cage, les diamètres d'ouverture de chaque ouverture présentant un ordre de grandeur de 0,5 mm à 0,7 mm et/ou les zones en forme de treillis dans la cage comportant des ouvertures ou des passages traversants avec des diamètres de 1,5 mm à 3,2 mm.
